# EUROPEAN PATENT APPLICATION

(11) **EP 4 710 974 A2**
(43) Date of publication of application: **18.03.2026**
(21) Application number: 25196873.1
(22) Date of filing: 19.08.2025
(51) Int. Cl.: A61M 16/01

(54) **MANIFOLDED ANESTHESIA AND VENTILATOR SYSTEM**

(30) Priority: 13.09.2024 US 202418885338
(71) Applicant: GE Precision Healthcare LLC, Waukesha, WI 53188 (US)
(72) Inventor: GILLING, Michael Andrew, Madison, 53718-6704 (US); CHRISTMAN, Thomas Aurele, Madison, 53718-6704 (US); KICKHOFEL, Rysa, Madison, 53718-6704 (US); RINDY, Ryan W., Madison, 53718-6704 (US)
(74) Representative: Kilburn & Strode LLP

(57) **Abstract**

Systems are provided for a manifolded anesthesia ventilator system (200). The manifolded anesthesia ventilator system (200) includes a core (201) and a kernel (1300). The core (201) includes a common gas manifold (204) and one or more module (203) and the kernel (1300) encases the core (203) and includes inputs (1308) and outputs (1309) fluidly coupled to the core (201) and is further coupled to a shell (1500).

## Description

### TECHNICAL FIELD

Embodiments of the subject matter disclosed herein relate to systems for a manifolded anesthesia ventilator system.

### BACKGROUND

An anesthesia and ventilator system may include a plurality of modules, such as an input module, a mixer module, a vaporizer module, and a breathing system. Conventionally, the modules may be coupled to each other in a series via tubing. The tubing may be a weak point of the anesthesia and ventilator system, prone to leaks and taking up a large amount of space within a housing of the anesthesia and ventilator system.

### BRIEF DESCRIPTION

In one embodiment, a manifolded anesthesia ventilator system comprises a core including a common gas manifold and one or more modules, and a kernel encasing the core and including inputs and outputs fluidly coupled to the core.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present invention will be better understood from reading the following description of non-limiting embodiments, with reference to the attached drawings, herein below:
FIG. 1 shows a schematic diagram of an anesthesia ventilator system of the prior art;
FIG. 2 shows a block diagram of a core of a manifolded anesthesia ventilator system, in accordance with one or more embodiments of the present disclosure;
FIG. 3 shows a schematic diagram of a first example of the core of the manifolded anesthesia ventilator system in a backbone configuration, in accordance with one or more embodiments of the present disclosure;
FIG. 4 shows a schematic diagram of a second example of the core in the backbone configuration, in accordance with one or more embodiments of the present disclosure;
FIG. 5 shows a first view of the core in a stacked configuration, in accordance with one or more embodiments of the present disclosure;
FIG. 6 shows a second view of the core in the stacked configuration, in accordance with one or more embodiments of the present disclosure;
FIG. 7 shows an example of a common gas manifold of the core, in accordance with one or more embodiments of the present disclosure;
FIG. 8 shows an example of an inlet module of the core, in accordance with one or more embodiments of the present disclosure;
FIG. 9 shows an example of a mixer module of the core, in accordance with one or more embodiments of the present disclosure;
FIG. 10 shows an example of a vaporizer module of the core, in accordance with one or more embodiments of the present disclosure;
FIG. 11 shows an example of a gas monitor module of the core, in accordance with one or more embodiments of the present disclosure;
FIG. 12 shows an example of a breathing system or the core, in accordance with one or more embodiments of the present disclosure;
FIG. 13 shows an illustration of the core included in a kernel of the manifolded anesthesia ventilator system, in accordance with one or more embodiments of the present disclosure;
FIG. 14 shows an illustration of the kernel included in a first shell of the manifolded anesthesia ventilator system, in accordance with one or more embodiments of the present disclosure; and
FIG. 15 shows an illustration of the kernel included in a second shell of the manifolded anesthesia ventilator system, in accordance with one or more embodiments of the present disclosure.

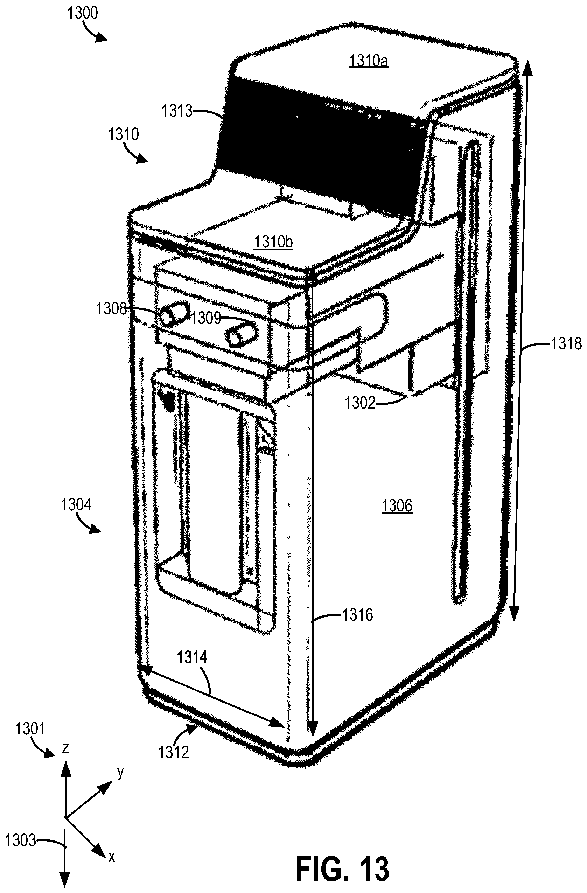

FIGS. 13-15 are shown approximately to scale, however, other dimensions may be used if desired.

### DETAILED DESCRIPTION

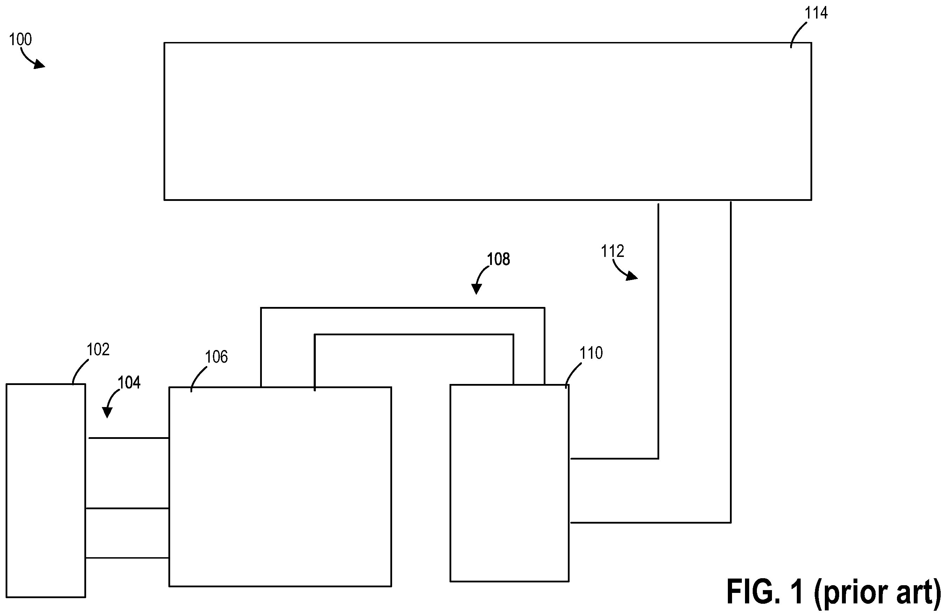

The following description relates to manifolded anesthesia ventilator systems. The manifolded anesthesia ventilator system may include a plurality of subsystems and/or modules, such as an inlet module, a mixer module, a vaporizer module, and a breathing system. Herein anesthesia ventilator system refers to a system capable of being used to deliver anesthesia and/or be used as ventilator. The manifolded anesthesia ventilator system may include a core which includes the subsystems and/or modules, a kernel encasing the core and providing user interfaces and optionally a shell further coupled to the kernel including additional features. Conventionally, as shown in FIG. 1, the subsystems/modules are configured in a distributed fashion, with each subsystem/module fluidly coupled in series via tubing. A flexibility of the tubing may facilitate positioning controls of the module such that they are accessible to an operator. The inventors herein have identified problems with the distributed system. The coupling points between the tubing and the modules are prone to leaking. Additionally, the tubing may take up a large amount of space, demanding a larger housing for the anesthesia ventilator system. Further, the distributed layout demands a large design and manufacturing effort to update the entire anesthesia ventilator system including to each module and subsystem as well as the housing if any modules are updated, removed or added.

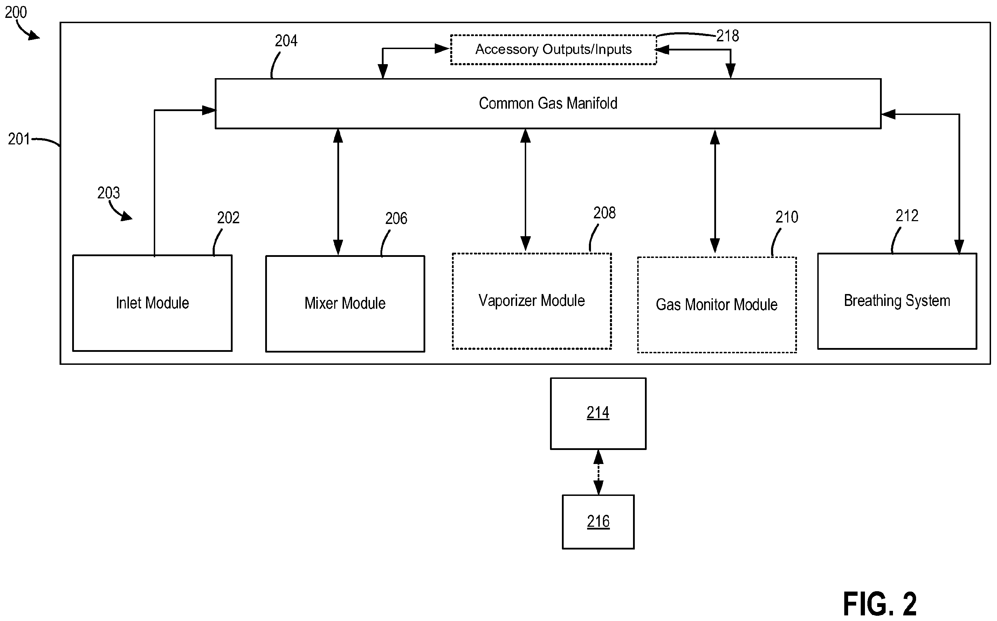

A manifolded anesthesia ventilator system may include a core positioned within a kernel and the kernel may be positioned within a shell. An example of the core is shown in the block diagram of FIG. 2. The core may include a common gas manifold configured to receive and distribute a plurality of gases (air, O₂, N₂O, and/or mixtures thereof) between the modules. Each module of the core may be directly fixedly coupled to the common gas manifold, thereby eliminating a demand for tubing to fluidly couple the modules. In this way, the system is in a modular configuration and updating, removing, and/or adding modules may be simplified. Further, the modules may couple directly to the manifold or may integrally formed with the manifold, reducing or substantially eliminating a demand for tubing. Additionally, each of the modules may be manufactured and leak tested separately before assembling the manifolded anesthesia ventilator system. When assembled, the leaks between the modules and common gas manifold may be tested. This process is simpler and easier to troubleshoot than assembling and testing the distributed anesthesia ventilator system with tubing as shown in FIG. 1.

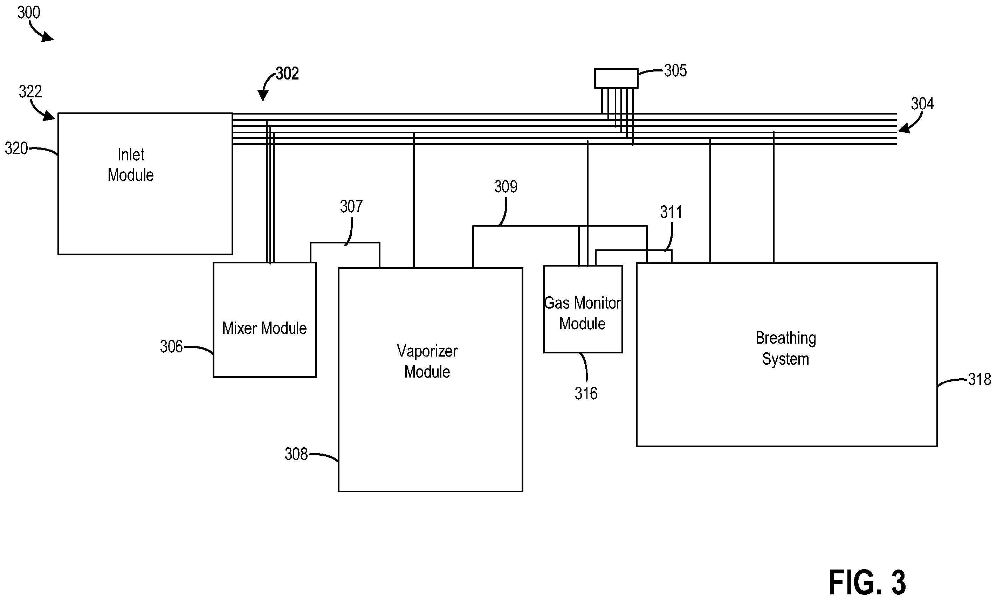

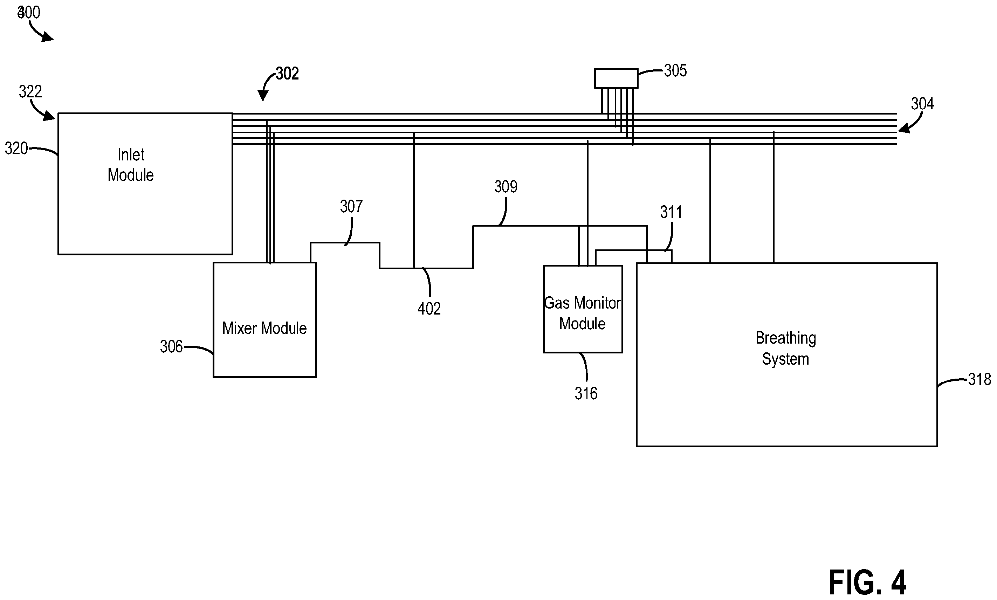

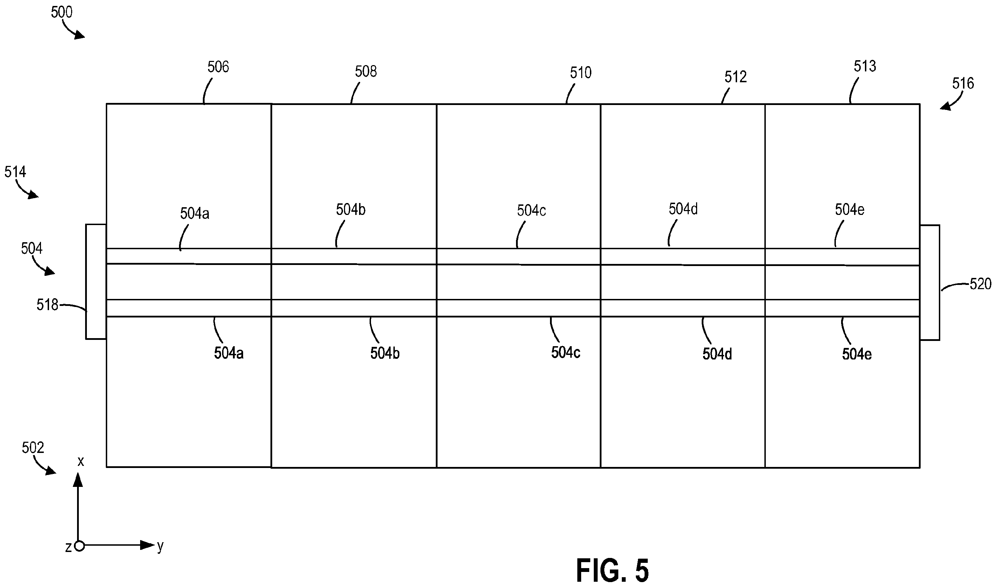

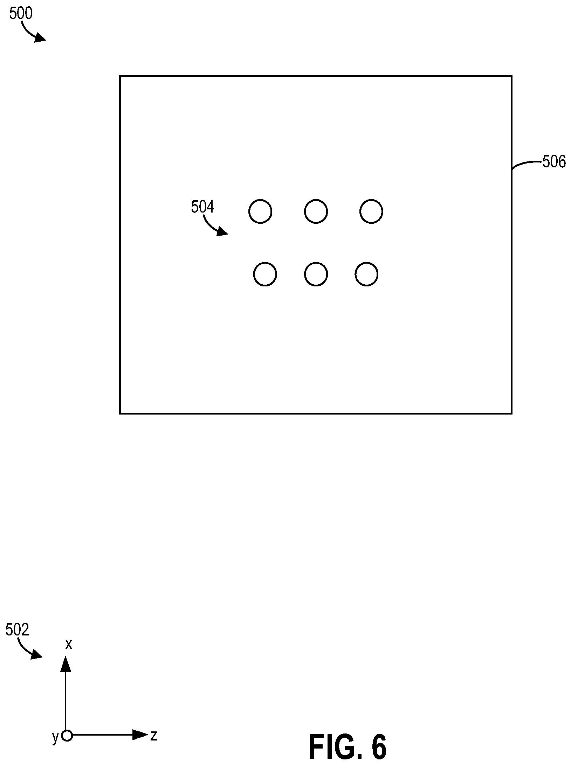

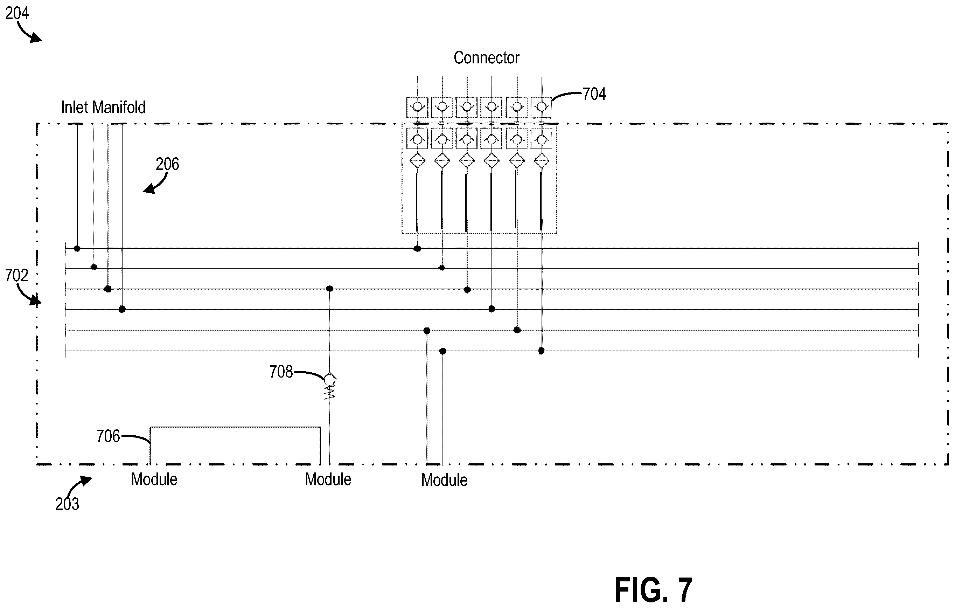

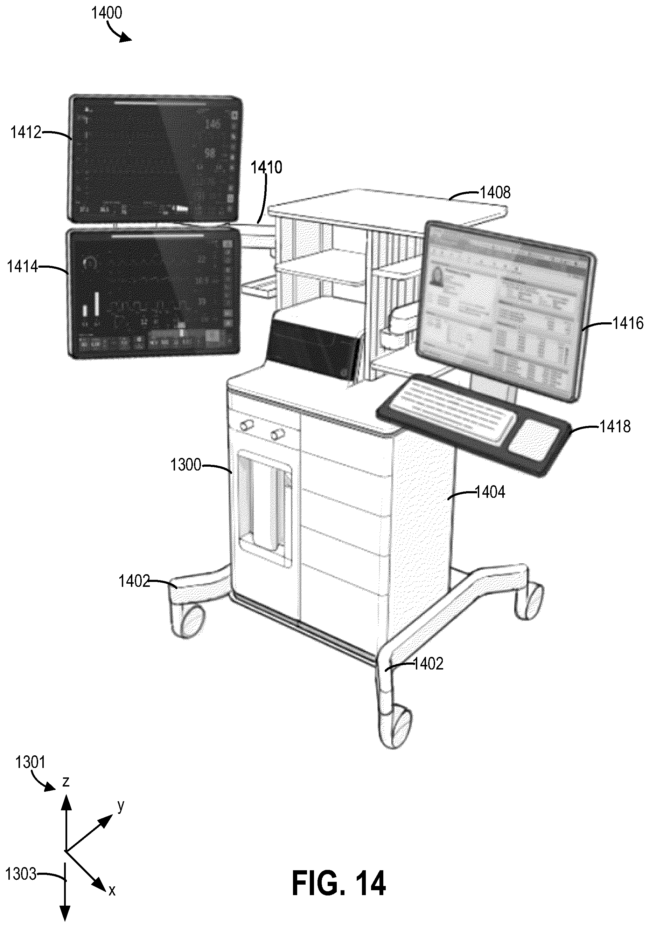

An example of the core in a backbone configuration is shown in FIG. 3. The common gas manifold may include a plurality of flow paths, through which gases may be passed between the different modules. As described above, the core of the manifolded anesthesia ventilator system may be easily adapted to different configurations, such as the alternate example of the core without a vaporizer module in FIG. 4. Alternatively, the core of the manifolded anesthesia ventilator system may be in a stacked configuration as shown in a cross-section view in FIG. 5 and perspective view in FIG. 6. Examples of modules included in the core are shown in FIGS. 7-12. The core of the manifolded anesthesia ventilator system may be incorporated into a kernel as shown in FIG. 13. The kernel may then be fitted into a plurality of different shells. The shells may vary based on a specific function of the manifolded anesthesia ventilator system or user preferences. Examples of the kernel positioned within different shells is shown in FIGS. 14-15.

Turning now to FIG. 1, a simplified example of an anesthesia ventilator system 100 of the prior art is shown. Anesthesia ventilator system 100 may include an inlet subsystem 102 fluidly coupled to a mixer subsystem 106 via a first tubing 104. As one example, first tubing 104 may include three different tubes, one each for N₂O, O₂, and air. A mixer subsystem 106 may then be fluidly coupled to a vaporizer subsystem 110 via a second tubing 108. Further, vaporizer subsystem 110 may be fluidly coupled to a breathing system 114 via third tubing 112.

Each of the tubing connections between the subsystems (e.g., mixer subsystem 106, vaporizer subsystem 110, etc.) may be a potential leak point in anesthesia ventilator system 100. Further, the tubing may take up a large amount of space, demanding a large housing. Additionally, the physical arrangement of the subsystems is limited by the tubing connections. If a subsystem is removed, anesthesia ventilator system 100 may demand adjustments to subsystems to which it is directly fluidly coupled. For example, removing vaporizer subsystem 110 may entail an adjustment to both of mixer subsystem 106 and breathing system 114. Removing the vaporizer subsystem 110 may also demand further rearrangement of physical controls of a housing of the anesthesia ventilator system 100.

Alternatively, a manifolded anesthesia ventilator system including a core comprising modules configured to be interchangeable and removable may at least partially overcome the challenges of the conventional design shown in FIG. 1. In one example, a common gas manifold for the gases of the anesthesia ventilator system may be provided. The core of the manifolded anesthesia ventilator system may be modular, whereby modules may be added, removed, and or updated without reconfiguring the whole system. Further, the physical, direct connections between the modules and the common gas manifold may be less prone to leaks than tubing connections. Even as the core is modularly updated, a kernel encasing the core may not be adjusted when the core is adjusted, thereby simplifying and streamlining manufacturing of different product lines using the core.

Turning now to FIG. 2, a block diagram of a manifolded anesthesia ventilator system 200 including a core of the manifolded anesthesia ventilator system 201 is shown. The core may include a common gas manifold 204 and one or more modules 203. As one example, the one or more modules may each be configured as manifolds to be coupled to common gas manifold 204. The one or more modules 203 may not include tubing. The one or modules 203 may include an inlet module 202 fluidly coupled to gas sources, and inlet module 202 may be configured to flow gas into common gas manifold 204. Common gas manifold 204 may distribute gases to a mixer module 206, and a breathing system 212. The core of the manifolded anesthesia ventilator system 201 may optionally include additional modules, such as but not limited to, vaporizer module 208 and gas monitor module 210. Each of the mixer module 206, breathing system 212 and optionally vaporizer module 208, and gas monitor module 210 may be fluidly coupled to each other and to the inlet module 202 via common gas manifold 204. The one or more modules 203 may each be directly fluidly coupled to common gas manifold 204. Further, Accessory outputs/inputs 218 which may be used in an anesthesia ventilator system, such as but not limited to suction, scavenging, auxiliary O₂, a high flow nasal cannula (HFNC), auxiliary common gas outlet (ACGO), intravenous medication pumps/controls, etc. may be fluidly coupled to common gas manifold 204. In this way, removing, adding, or updating a module of the core of the manifolded anesthesia ventilator system may demand a small modification to a single module and not updates to other components of the manifolded anesthesia ventilator.

The manifolded anesthesia ventilator system 200 may further include a controller 214. Controller 214 may be communicatively coupled, either wired or wirelessly, to pneumatic actuators, valves, sensors, and such of the core of manifolded anesthesia ventilator system 201. Controller 214 may include a central processor (CPU) or other electronic components capable of carrying out processor functions for operation of the one or more modules and common gas manifold manifold of the core. Controller 214 may be communicatively coupled, either wired or wirelessly, to a user interface 216. In this way, a user may control the actuators and valves of the core of manifolded anesthesia ventilator system 201 from wherever user interface 216 may be positioned. For example, user interface 216 may include a keyboard and mouse. Additionally or alternatively, user interface 216 may include a touch screen or physical buttons positioned on a housing, or graphical controls of an application run on a physically separate smartphone or tablet. In this way, physical positioning of the actuators to control the one or more modules of the core of manifolded anesthesia ventilator system 201 may be accessed by the user from any location and positioning of modules within the manifolded anesthesia vaporizer system may not be based on a demand that pneumatic controls be physically accessible to a clinician.

Turning briefly to FIGS. 7-12, diagrams of non-limiting examples of common gas manifold 204 and one or more modules 203 are shown. The diagrams of FIGS. 7-12 include a plurality of valves and other actuators which may be communicatively coupled to a controller of the manifolded anesthesia ventilator system, such as controller 214. FIG. 7 shows a non-limiting example of common gas manifold 204. Common gas manifold 204 may include a plurality of flow paths 702. The plurality of flow paths may each be independent flow paths. In some examples, each of the plurality of flow paths may be parallel flow paths. Herein flow paths refs to how gas is flowed and not physical positioning of the manifold. Herein, parallel flow paths refers to flow paths wherein each flow path is an independent flow path and gas flowing through each flow path does not mix within the other gases within the parallel flow path. The physical manifolds through which the gases flow may or may not be parallel. Each flow path may be dedicated to a type of gas used in the manifolded anesthesia ventilator system. For example, common gas manifold 204 may include six flow paths. The six flow paths may be individually configured to flow O₂, N₂O, air, drive gas, scavenging gas, and pressure regulated O₂. Other numbers of flow paths included in the common gas manifold are also considered within the scope of the present disclosure. Each flow path may be parallel to the others and may not be directly fluidly coupled to each other. In some examples, a physical length of each flow path may be equivalent.

Common gas manifold 204 may include a connector port 704. Connector port 704 may be a single port containing a plurality of pneumatic and electrical connections. Connector port 704 may provide fluid connections to each of the flow paths of common gas manifold 204. As one example, connector port 704 may be used if any of the gases of the manifolded anesthesia ventilator system are to be coupled to an additional accessory component. Further, connector port 704 may be used to directly couple common gas manifold 204 a kernel and optionally a shell of the manifolded anesthesia ventilator system.

Modules 203 may be fluidly coupled to common gas manifold 204. In some examples, common gas manifold may include a connection path 706 which couples the output of one module to an input of another module. In further examples such as inlet module 202, modules may be directly coupled to flow paths 702. In some examples, common gas manifold 204 may include a valve 708 positioned in a flow path between a module and a flow path of flow paths 702.

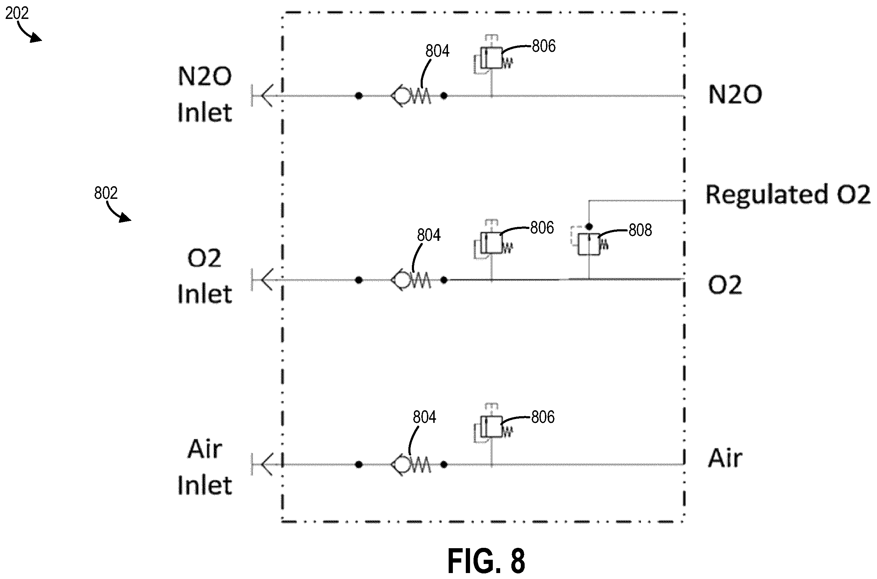

Turning now to FIG. 8, a non-limiting example of inlet module 202 is shown. Inlet module 202 may be physically coupled to an upstream end of the common gas manifold, as shown in FIG. 7. In an alternate example, inlet module 202 may be integrally formed as part of common gas manifold 204. In such an example, inlet module 202 may not be readily decoupled from common gas manifold 204. Inlet module may include inlets 802 to introduce O₂, air, and N₂O to the designated flow channels of common gas manifold 204. In some examples, inlet module may not include an N₂O inlet. In some examples, other gases instead of or in addition to N₂O may be introduced to common gas manifold 204 by inlet module 202. In some examples, inlet module 202 may be coupled to respective gas cylinders and/or to in-house supplies of O₂, air, and N₂O. In some examples, inlet module 202 may be configured to combine O₂ and air to be coupled to a drive gas flow channel of common gas manifold 204.

Inlet module 202 may include pressure relieve valves 804 positioned in the fluid path between the inlets 802 on the outputs to the common gas manifold 204. Inlet module 202 may further include pressure regulators 806 positioned between the pressure relieve valves 804 and the outputs to the common gas manifold 204. Pressure regulators 806 may reduce a pressure receives from inlets 802 to a useable pressure. In some examples an O₂ fluid path may be branched to an O₂ outlet and a regulated O₂ output. A second pressure regulator 808 may be positioned between the main O₂ fluid path and the regulator O₂ output.

In some examples, an inlet module may include different types or a different number of gases. In some examples, inlet module 202 may be replaced with the different inlet module having a different number of gases or different types of gases. Replacing inlet module 202 with the different inlet module may not demand modifications to common gas manifold 204 or other modules of the core of manifolded anesthesia ventilator system 201.

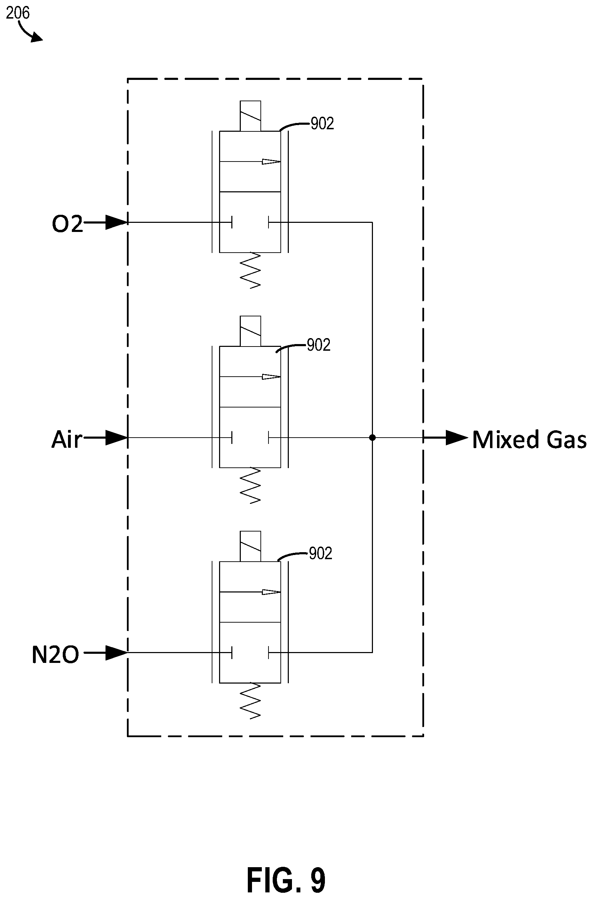

Turning now to FIG. 9, a non-limiting example of mixer module 206 is shown. Mixer module 206 may be coupled directly to the N₂O, air, and O₂ flow paths of common gas manifold 204. In this way, mixer module 206 may receive the gases to be mixed via common gas manifold 204 and not by coupling directly to the inlet module 202. Mixer module 206 may output mixed gases in a single output back to common gas manifold 204 via additional flow channels or connection channels, such as connection path 706. In some examples, the flow path for N₂O may not be included and O₂ and air may be combined without N₂O. In alternate examples N₂O may be replaced with a different gas. Mixer module 206 may include flow regulators 902. Each of the gas inlets received may be directed to one of flow regulators 902 and outputs from flow regulators 902 may then be combined to output a single mixed gas output. Flow regulators may be used to control a flow rate of each gas to the common mixed gas output, thereby controlling a composition of the mixed gas.

In one example, mixer module 206 may be configured as a first mixer module or a second mixer module. For example, the first mixer module may be a high flow mixer module and the second mixer module may be a low flow mixer module. The first mixer module and the second mixer module may be interchangeable with respect to core 201 of manifolded anesthesia ventilator system 200. In one example portions of first mixer module and second mixer module coupling to common gas manifold 204 may both be formed in the same way, while the mixing components may be replaced depending on the demanded flow rate. In this way, mixer modules may be replaced or updated with mixer manifolds having different configurations to produce different manifolded anesthesia ventilator system products without updating common gas manifold 204 or other modules of the manifolded anesthesia ventilator system core.

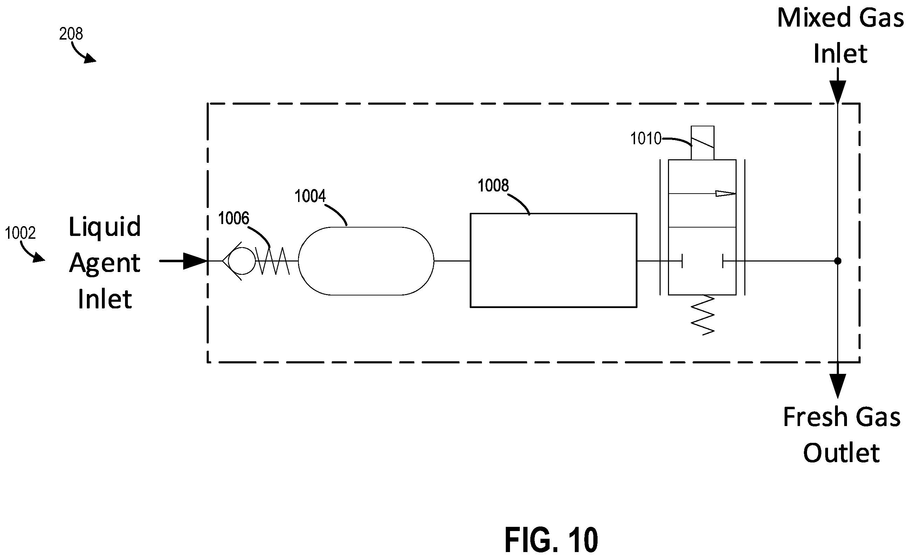

Turning now to FIG. 10, a non-limiting example of optional vaporizer module 208 is shown. Vaporizer module 208 may be positioned downstream of mixer module 206. Vaporizer module 208 may include an input 1002 for liquid anesthetic agent. Liquid anesthetic agent may flow to a storage tank 1004. A valve 1006 may be positioned in a flow path between input 1002 and storage tank 1004. Storage tank 1004 may be fluidly coupled to a liquid gas converter 1008. Liquid gas converter 1008 may convert liquid anesthetic agent to gaseous anesthetic agent. For example, liquid gas converter 1008 may include a heater to evaporate the liquid anesthetic agent to the gas phase. As another example. liquid gas converter may include a pressurized injector to convert the liquid anesthetic agent to the gas phase. The gaseous anesthetic agent may flow from liquid gas converter 1008 to a flow path downstream of mixed gas (e.g., output by mixer module 206). A flow controller 1010 may be positioned between the output of liquid gas converter 1008 and the stream of mixed gas. The flow controller may control the amount of gaseous anesthetic mixed into the mixed gas. Mixed gas mixed with gaseous anesthetic agent may be referred to as fresh gas. Vaporizer module 208 may receive a mixed gas input and output fresh gas.

In some examples, such as when anesthesia drugs are provided intravenously, vaporizer module 208 may not be used and the core of the manifolded anesthesia ventilator system may be adapted accordingly without modifying the common gas manifold or other modules, as described further below with respect to FIG. 4.

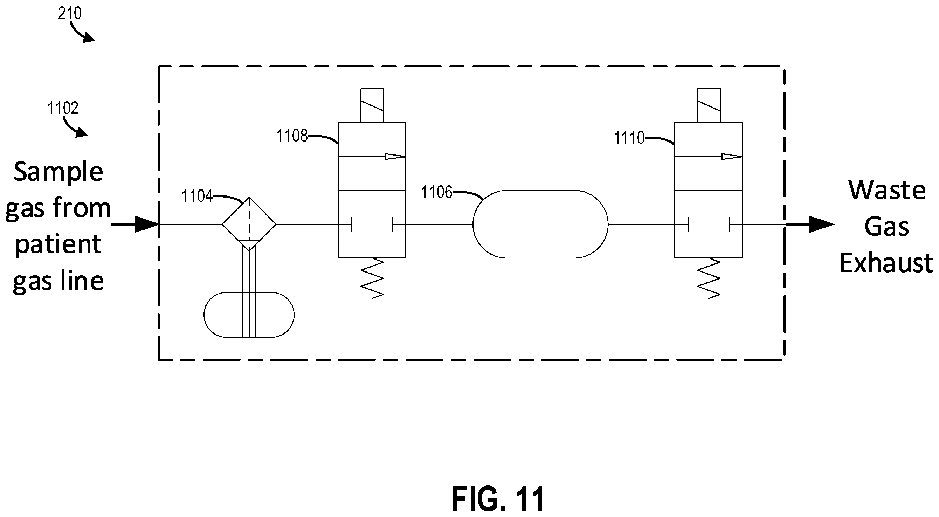

Turning now to FIG. 11, a non-limiting example of gas monitor module 210 is shown. Gas monitor module 210 may receive a sample gas inlet 1102 from a patient line. The patient line may be an output of a breathing system, such as breathing system 212. The sample gas may flow to a water separator 1104. Water separator 1104 may condense and trap any water vapor in the sample gas. The sample gas may then flow to gas analyzer 1106. Gas analyzer 1106 may determine a concentration of O₂, CO₂, and anesthetic agent in the sample gas. An inlet flow controller 1108 may be positioned between water separator 1104 and gas analyzer 1106. Inlet flow controller may control a flow rate of sample gas introduced to gas analyzer 1106. An outlet flow controller 1110 may be positioned downstream of gas analyzer 1106 and may control a flow of analyzed sample gas output by gas analyzer 1106 after analysis. Gas monitor module 210 may output waste gas. In some examples waste gas may be recirculated to the breathing system. In alternate examples, waste gas may be output to a scavenging system.

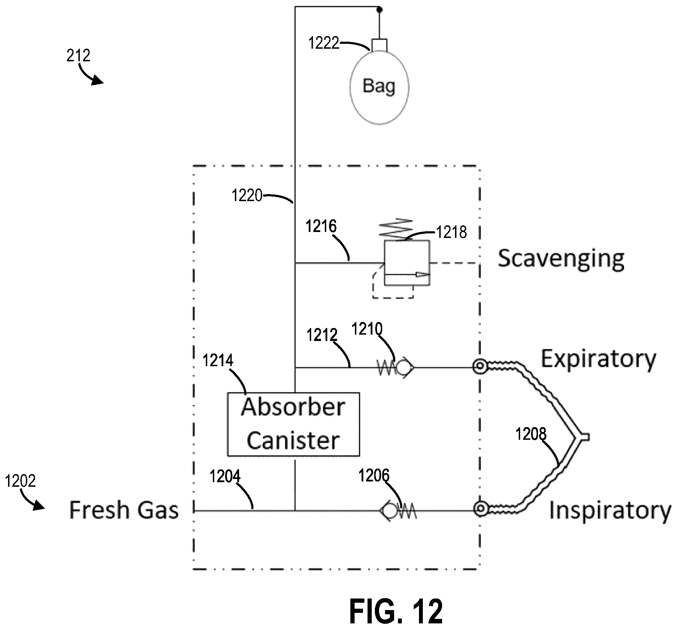

Turning now to FIG. 12, a non-limiting example of breathing system 212 is shown. Breathing system may include a fresh gas input 1202. As discussed above, fresh gas input may be output by a vaporizer module, such as vaporizer module 208. Fresh gas may be directed to an input flow path 1204 and flow to an inspiratory arm of a patient coupler 1208. A valve 1206 may be positioned in input flow path 1204 to control a direction of flow into the inspiratory arm. Gas exhaled by the patient may pass into an expiratory arm of patient coupler 1208 and into an output flow path 1212. A valve 1210 may be positioned in output flow path 1212 to control a direction of flow out of the expiratory arm. In some example gas exhaled by the patient may be directed to an absorber 1214 to absorb exhaled CO₂ and then back to input flow path 1204. Excess gas not directed back to input flow path 1204 may be directed to waste flow path 1216 and to a scavenging system. In some examples, gas exhaled by the patient may not be recycled and the expiratory arm may be directly coupled to waste flow path 1216. Waste flow path 1216 may include a pressure regulator 1218 positioned upstream of the outlet to the scavenging system to control a pressure of gas introduce to the scavenging system. A driver flow path 1220 may fluidly couple each of input flow path 1204, output flow path 1212, and waste flow path 1216 and may be coupled to a gas driver to propel the gas to and from the patient. In one example, driver flow path 1220 may be coupled to a bag 1222. In alternate examples, driver flow path 1220 may be coupled to bellows and may include valves to control a direction of gas flow.

One example of a core 300 of the manifolded anesthesia ventilator system 200 is shown in FIG. 3. Core 300 may be an example of the core of manifolded anesthesia ventilator system 201 shown in FIG. 2. Core 300 may be an example of a core in a backbone configuration (e.g., a backbone core 300).

In the backbone configuration the common gas manifold may act as a backbone and the one or modules of the core of the manifolded anesthesia ventilator system may each be directly fixedly coupled to sides of the common gas manifold and the common gas manifold may be a continuous manifold that is separable from and spaced away from the one or more modules. Directly fixedly coupling may include, but is not limited to, bolting, clamping, magnetically securing, other mechanical fasteners capable of maintaining fluid-tight connections, and gluing. Common gas manifold 302 may be an example of common gas manifold 204 shown in FIG. 2. Gas may flow through common gas manifold 302 from an upstream end 322 to a downstream end 304. Common gas manifold 302 may include a connector port 305. Connector port 305 may be an example of the connector port of common gas manifold 204 shown in FIG. 7.

An inlet module 320 may be coupled to upstream end 322 of common gas manifold 302. Inlet module 320 may be an example of inlet module 202 of FIGS. 2 and 8. A mixer module 306 may be coupled to common gas manifold 302 down stream of inlet module 320. Mixer module 306 may be an example of mixer module 306 of FIGS. 2 and 9 and may receive gases from inlet module 320 via common gas manifold 302 and may output mixed gas to vaporizer module 308 by a first connection path 307. Vaporizer module 308 may be an example of vaporizer module 208 of FIGS. 2 and 10. Vaporizer module 308 may be coupled directly to a flow path of common gas manifold 302 downstream of mixer module 306. Fresh gas output by vaporizer module 308 may be output to gas monitor module 316 and breathing system 318 via a second connection path 309. Gas monitor module 316 may be an example of gas monitor module 210 of FIGS. 2 and 11. Gas monitor module 316 may receive sample gas from breathing system 318 via third connection path 311. Gas monitor module 316 may also be directly coupled to a flow path of common gas manifold 302. Breathing system 318 may be an example of breathing system 212 of FIGS. 2. and 12. Breathing system 318 may be directly coupled to a downstream end 304 of common gas manifold 302 in addition to being coupled to gas monitor module 316 and vaporizer module 308 via third connection path 311 and second connection path 309 respectively.

As described above, core 300 in the backbone configuration may include one or more modules (e.g., a plurality of modules) bolted to common gas manifold 302. Each of the modules performs a separate function of the manifolded anesthesia ventilator system and may be interchanged or updated with different modules performing the function. Further, a demand for pneumatic tubing running between the plurality of modules is avoided, thereby saving space and eliminating the potential leak points introduced by the pneumatic tubing connections. Another advantage of a manifolded anesthesia ventilator system core, such as core 300, is that a manufacturer may readily add and/or remove modules as demanded, to produce one or more products of a product line, each product including at least the same common gas manifold.

As one example, core 300 may be easily converted to an intravenous anesthesia core 400, as shown in FIG. 4. Intravenous anesthesia core 400 may include some of the same components as core 300, such components are numbered the same and are not reintroduced. Intravenous anesthesia may use intravenous drugs to anesthetize the patient instead of vaporized gases. For this reason, manifolded intravenous anesthesia ventilator system may not include a vaporizer module, such as vaporizer module 308.

To transform core 300 into intravenous anesthesia core, the vaporizer module may be removed while the common gas manifold and remaining modules are unchanged. In one example, the vaporizer module may be replaced by a module coupler 402. Module coupler 402 may be single flow path bolted at a first end to first connection path 307 and at a second end to second connection path 309 in the same physical position as vaporizer module 308. Module coupler 402 may also include a cap terminating the flow path which had coupled the vaporizer module to common gas manifold 302. In this way, mixer module 306 may still be coupled to breathing system 318 through module coupler 402. The module coupler may be a flow path between open flow paths when a module is removed. Module coupler 402 may act as a pathway though which gases flow and may not include pneumatic components or valves. Replacing the vaporizer module with module coupler 402 may be an efficient way to adapt core 300 into intravenous anesthesia core 400. Comparing core 300 of FIG. 3 with intravenous anesthesia core 400 of FIG. 4, other components including the other modules and the common gas manifold may be substantially the same and unchanged.

In alternate examples, core 300 may be adapted in different ways by the manufacturer depending on demands of the user. Modules may be removed and replaced with module couplers and/or endcaps to maintain fluid connectivity of the remaining modules throughout. Modules may also be easily replaced and/or updated to prevent degradation of the manifolded anesthesia ventilator system. In some examples a second common gas manifold may be coupled to the common gas manifold 302 to extend the core 300 and add further modules to the core. In some examples, the second common gas manifold may be coupled to common gas manifold 302 via connector port 305.

Turning now to FIGS. 5 and 6, an illustration of an alternate example of a core 500 is shown. Core 500 may be an example of a stacked configuration core. A reference axis 502 is included for the comparison of the views in FIG. 5 and FIG. 6, including an x-axis, y-axis, and z-axis. The y-axis may be parallel to a longitudinal axis of core 500 and parallel to a direction of flow of gases through core 500. The x-axis may be parallel to a lateral direction of core 500. FIGS. 5 and 6 show a simplified illustration of the core 500. Modules of core 500 may include the same flow paths and other components as those shown in FIGS. 7-12. Core 500 may also include connection flow paths to couple modules which are also not shown in FIGS. 5-6.

FIG. 5 is a cross sectional view of core 500 showing two flow paths of a common gas manifold 504. Common gas manifold 504 may be an example of common gas manifold 204 of FIG. 2. Common gas manifold 504 may include six flow paths as described above with respect to common gas manifold 302. The cross sectional view of FIG. 5 therefore shows two of the six flow paths. Other arrangements of flow paths are also considered and are described further below with respect to FIG. 6. Common gas manifold 504 may be separated into a plurality of different sections which may be integrated into the stacked modules. In one example, the common gas manifold may be separated into five different sections: first section 504a, second section 504b, third section 504c, fourth section 504d, and fifth section 504e. Each of the plurality of sections may be integrally formed into a module of core 500. A face of each module in the x-z plane may be configured to be bolted to a neighboring module. When the modules are bolted together (e.g., affixed in face-sharing contact), the respective sections of the common gas manifold align, thereby forming flow paths to carry gases between the modules.

First section 504a may be integrally formed into inlet module 506 and comprising an upstream end 514 of core 500. Mixer module 508 may be bolted to a downstream end of inlet module 506. Second section 504b of common gas manifold 504 may be integrally formed into mixer module 508 and may align with first section 504a when the two are bolted together. Vaporizer module 510 may be bolted to a downstream end of mixer module 508. Third section 504c may be integrally formed into vaporizer module 510. Gas monitor module 512 may be bolted to a downstream end of vaporizer module 510. Fourth section 504d of common gas manifold 504 may integrally formed into gas monitor module 512. Breathing system 513 may be bolted to a downstream end of gas monitor module 512. Fifth section 504e of common gas manifold 504 may be integrally formed into breathing system. Sections of common gas manifold 504 integrally formed into respective modules may form a continuous flow path for gases when modules of core 500 are coupled together.

Modules of core 500 may be added, removed, reconfigured, and/or replaced depending on modules demanded for the anesthesia ventilator system. For example, manifolded anesthesia ventilator system may be modified to not have a vaporizer module or to have multiple breathing systems.

Core 500 may further include an upstream end cap 518 positioned at the upstream end 514 of common gas manifold 504. Upstream end cap 518 may seal the upstream end of common gas manifold 504 from leaks. Core 500 may further include a downstream end cap 520 positioned at downstream end 516 of common gas manifold 504. Downstream end cap 520 may seal the downstream end of common gas manifold 504 from leaks. In some examples, downstream end cap 520 may include accessory outputs and inputs (e.g., accessory outputs and inputs 218) fluidly coupled to common gas manifold 504, such as a patient gas sample, high flow nasal oxygen, or auxiliary common gas outlet.

Turning now to FIG. 6, a view of core 500 is shown looking along the y-axis through either an upstream end or downstream end, through upstream end cap 518 or downstream end cap 520. The view of FIG. 6 shows the six flow paths of common gas manifold 504. The spatial arrangement of the flow paths of common gas manifold 504 in the x-z plane may not be particularly limited. In one example the spatial arrangement of flow paths of common gas manifold 504 may be the same in each module of core 500. In an alternate example, the spatial arrangement of flow channels may be varied and connections between each module may be different. The spatial arrangement of the flow paths of common gas manifold 504 may be in a circular arrangement or in a single column along the x or z axis. Additionally, flow paths of common gas manifold 504 may have equivalent diameters, as shown in FIG. 6, or the flow paths may be formed with different diameters. Further, although shown as circles herein, other shapes of flow baths are also considered.

Core 300 and intravenous anesthesia core 400 may comprise a first product and second product of a product line. The product line may include components having common or overlapping features as well as differences. For example, a common gas manifold comprising a plurality of flow paths fluidly coupling modules of the core of the manifolded anesthesia ventilator system may be a common feature of the product line. Products of the product line may include different modules directly fluidly coupled to the common gas manifold. For example, the first product (e.g., core 300) may include an inlet module, mixer module, vaporizer module, gas monitor module, and breathing system. The second product (e.g., intravenous anesthesia core 400) may include the same inlet module, mixer module, gas monitor module, and breathing system as the first product and may also include a module coupler in place of the vaporizer module.

In a backbone configuration, the common gas manifold may be formed as one continuous part on which modules are bolted, as shown in FIGS. 3 and 4. In a stacked configuration, the common gas manifold may be manufactured in separate sections which form continuous flow paths by bolting modules together, wherein each module comprises a section of the common gas manifold arranged to align to form the common gas manifold as shown in FIGS. 5 and 6. The first and second product as described above may be both be in backbone configuration or both in the stacked configuration.

A manifolded anesthesia ventilator system may comprise a core such as the cores shown in FIGS 2-6. The manifolded anesthesia ventilator system may further include additional components such as a housing, accessory components, and user interfaces. The core of the manifolded anesthesia ventilator system may be incorporated into a kernel. The kernel may be configured to be integrated with any core of the protect line. For example, modules of the core may be added, removed, and or interchanged without changing the kernel. The kernel may be fitted into a shell of the manifolded anesthesia ventilator system. The shell may include accessories that may be selected based on a use case of the manifolded anesthesia ventilator system. As described above, modules of the manifolded anesthesia ventilator system may be added and removed based on demands of the device, without changing the kernel. Such adjustments may not be possible if modules are coupled via tubing, where adding/removing modules may demand significant repositioning of each component. In this way, a plurality of different anesthesia ventilator devices may be constructed as desired based on demands of the user, without having to significantly change the core or the kernel. Manufacturing may benefit from economies of scale by manufacturing a larger number of core housings configured to add on customizable components as demanded by the user. Examples of manifolded anesthesia ventilator systems, each of which may a core are shown and discussed further below with respect to FIGS. 13-15. A reference axis 1301 including an x-axis, y-axis, and z-axis is provided for comparison of FIGS. 13-15. The x-axis may be parallel to a lateral axis and the y-axis may be parallel to a longitudinal axis. The z-axis may be parallel to vertical axis.

Turning now to FIG. 13, an illustration of a kernel 1300 is shown. The view of kernel 1300 shown in FIG. 13 is partially transparent to show an outline of a core 1302 positioned within and encased by kernel 1300. Core 1302 may be an example of cores discussed above with respect to FIGS. 2-6. Kernel 1300 may be configured to couple to any core of a product. In this way, modules of core 1302 may be changed while kernel 1300 remains the same.

Kernel 1300 may be roughly shaped as rectangular prism including a front face 1304 and a rear face positioned opposite front face 1304 across the y-axis. Kernel 1300 may further in include a first side 1306 and a second side positioned opposite first side 1306 across the x-axis. First side 1306 and the second side may extend from front face 1304 to the rear face. Front face 1304 may include an input 1308 and output 1309. Input 1308 and output 1309 may couple to inspiratory and expiratory portions of a patient coupler of a breathing system, such as patient coupler 1208 of breathing system 212 shown in FIG. 12. Front face 1304 may further include a user interface 1313. User interface 1313 may be an example of user interface 216 shown in FIG. 2. In some examples, user interface 1313 may be a touch screen and may also function as a display. The rear face may include system connections of the manifolded anesthesia ventilator system. Systems connections may include one or more gas connections, power connections, and communications connections. In one example, connection may be made through a connector port of the common gas manifold, such as connector port 704. First side 1306 and the second side may be configured to couple to demanded accessories of the manifolded anesthesia ventilator system or to components of the shell.

Kernel 1300 may further include a top face 1310 and a bottom face 1312. Top face 1310 and bottom face 1312 may be opposite each other across the z-axis. Top and bottom may herein refer to relative positions with respect to a direction of gravity as indicated by arrow 1303. Bottom face 1312 may be roughly shaped as rectangle. Top face 1310 may be divided into a first section 1310a and a section 1310b divided by user interface 1313. Both top face 1310 and bottom face 1312 may be configured to be coupled to accessories of the manifolded anesthesia ventilator system.

Kernel 1300 may be a width 1314. Width 1314 may be slightly (e.g., 5%) wider than a maximum width of core 1302. Width 1314 may be slightly wider than a width of two size E gas cylinders. Kernel 1300 may be a first height 1316 at front face 1304 and may slope upwards to reach a second height 1318 at the rear face. First height 1316 may be less than second height 1318. Second height 1318 may be slightly higher than a height of a size E gas cylinder. A vertical positioning of input 1308, output 1309 and display 1312 may be at proven heights for anesthesia ventilator systems.

Kernel 1300 may be laterally symmetric (e.g., symmetric across the z-y plane) In this way coupling of accessories to kernel 1300 may be easily done for left handed or right handed placement and/or use. For example, if a user interface is further coupled to kernel 1300, it may be coupled to either first side 1306 or the second side on the same kernel 1300. In some examples, the anesthesia manifold system may comprise kernel 1300 without any further shell. Such an example may be used where space is limited, such as in an operating room. Accessory inlets/outlets coupled to the core may include patient interface positioned on kernel 1300. For example, kernel 1300 may include additional patient connections, ACGO, scavenging, suction, auxiliary O₂, and HFNC. Other accessories may also be coupled directly coupled to kernel 1300. Accessories may include, but are not limited do, supplementary vaporizers, patient connections, CO₂ canister, clinical controls, displays, structural connections, work surfaces, and a bag arm.

In some examples a kernel, such as kernel 1300 of FIG. 13 may be incorporated into a shell. An example of kernel 1300 incorporated into a first shell 1400 is shown in FIG. 14. First shell 1400 may include casters 1402 coupled to the bottom surface of kernel 1300. First shell 1400 may further include drawers 1404 coupled to the first side of kernel 1300. In alternate examples drawers may be coupled to the second side of kernel 1300. First shell 1400 may further include a shelf unit 1408 coupled to top portions of the first side and second side of kernel 1300 and extending above the top face of kernel 1300. Shelf unit 1408 may include one or more shelves. Sides of shelf unit 1408 may parallel with the first side and second side of kernel 1300 and may be coupled to arms configured hold displays. A first arm 1410 may be coupled to a first side of shelf unit 1408 and may support a first display 1412 and a second display 1414. A second arm may extend from second side of shelf unit 1408 and may support a third display 1416 and a user interface 1418. Positions of first arm 1410 and the second arm may be swapped to provide an example of the shell where lateral positions (e.g., along the x-axis) or first display 1412, second display 1414, third display 1416, and user interface 1418 may be swapped. In this way, components of the shell, such as first shell 1400, may be laterally interchangeable with respect to the first side and second side of kernel 1300.

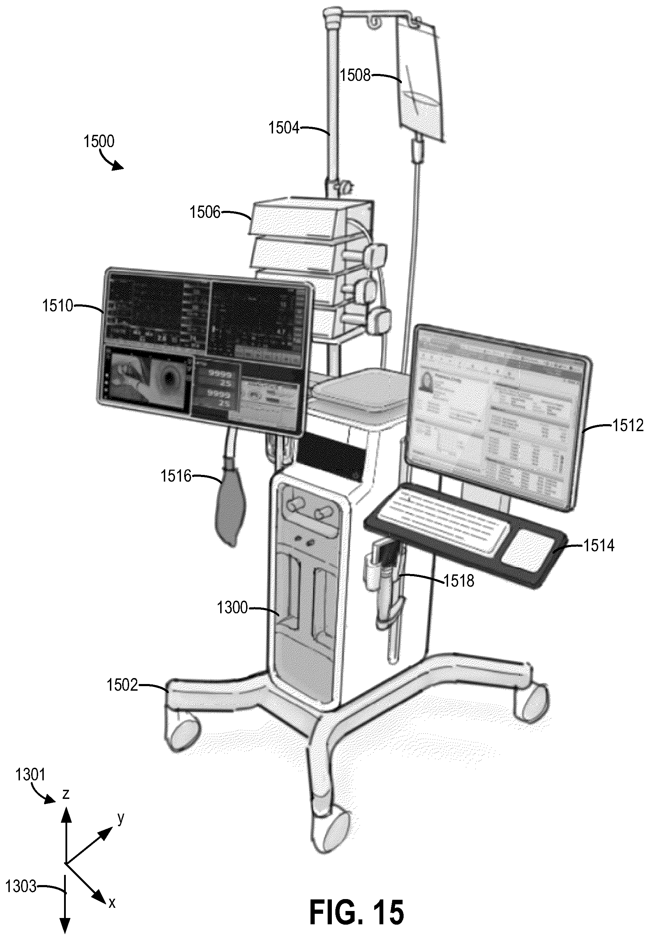

Turning now to FIG. 15, an example is shown of kernel 1300 incorporated into a second shell 1500. Second shell 1500 may be included in manifolded anesthesia ventilator system set up for intravenous anesthesia. For example, the core included in kernel 1300 may not include a vaporizer module. Second shell 1500 may include casters 1502 coupled to the bottom surface of kernel 1300. Second shell 1500 may conform to a width, height and depth of kernel 1300. Shell 1500 may include a pole 1504 extending vertically above the top face of kernel 1300. Pumps 1506 may be coupled to pole 1504. Pole 1504 may also support an IV bag 1508. Pumps 1506 may deliver intravenous anesthetic agent to IV bag 1508 which is then introduced to the patient. Second shell 1500 may further include a first display 1510 coupled to a second side of second shell 1500 and. A bag arm 1516 may also be coupled to second side of second shell 1500. Bag arm 1516 may be fluidly coupled to the breathing system included the core. A second display 1512 and a user interface 1514 may be further coupled to a first side of second shell 1500. The first side of second shell 1500 may further include a holder 1518 configured to hold medical equipment to be used the clinician. For example, holder 1518 may be configured to hold a laryngoscope.

The technical effect of the manifolded anesthesia ventilator systems described herein is to help reduce an occurrence of fluid leaks by replacing tubing with a common gas manifold coupled to one or more modules. The modules may be bolted to each other and/or the common gas manifold in manner which is more physically secure and saves space compared to the use of tubing. Further, modules of the manifolded anesthesia ventilator systems using the common manifold may be easily removed, added, updated to form different products of a product line, while still reusing many parts between the different products. In this way, manufacturing advantages of increased product quantities may be maintained while still providing a range of products based on user demands. Further, the manifolded anesthesia ventilator system may be comprise a core of a manifolded anesthesia ventilator system and additional features and/or modifications may be positioned around the core in a kernel, where the kernel may be adjusted without adjusting the core, further additional features may be added in a shell that is coupled to the kernel, thereby providing further product scale advantages in manufacturing the core and kernel.

The disclosure also provides support for a manifolded anesthesia ventilator system, comprising: a core including a common gas manifold and one or more modules, a kernel encasing the core and including inputs and outputs fluidly coupled to the core. In a first example of the system, the common gas manifold includes a plurality of flow paths and the one or more modules include an inlet module, mixer module, and breathing system. In a second example of the system, optionally including the first example, the one or more modules further include one or more of a vaporizer module and gas monitor module. In a third example of the system, optionally including one or both of the first and second examples, the system further comprises: a shell coupled to the kernel, wherein the shell includes one or more of a shelf unit, drawers, a display, and a user interface. In a fourth example of the system, optionally including one or more or each of the first through third examples, the core is in a backbone configuration and the common gas manifold is spaced away from the one or more modules. In a fifth example of the system, optionally including one or more or each of the first through fourth examples, the core is in a stacked configuration and the common gas manifold is integrally formed in the one or more modules. In a sixth example of the system, optionally including one or more or each of the first through fifth examples, the kernel is laterally symmetric and components of a shell coupled to the kernel may be laterally interchangeable with respect to sides of the kernel. In a seventh example of the system, optionally including one or more or each of the first through sixth examples, a width of the kernel may be slightly wider than a width of two size E gas cylinders and a height of the kernel is slightly higher than a height of a size E gas cylinder.

The disclosure also provides support for a product line of a manifolded anesthesia ventilator system, the product line comprising: a first product comprising an inlet module, a mixer module, a vaporizer module, a gas monitor module, and a breathing system, each of the inlet module, the mixer module, the vaporizer module, the gas monitor module, and the breathing system fluidly coupled to a common gas manifold, and a second product comprising the inlet module, the mixer module, the gas monitor module, a module coupler, and the breathing system, each of the inlet module, the mixer module, the module coupler, the gas monitor module and the breathing system fluidly coupled to the common gas manifold, wherein the common gas manifold is comprised of a plurality of flow paths, and wherein each of the plurality of flow paths are configured to direct a gas or mixture of gases. In a first example of the system, a position of the vaporizer module on the common gas manifold in the first product is the same as a position of the module coupler on the common gas manifold in the second product. In a second example of the system, optionally including the first example, the gas or mixture of gases includes one or more of oxygen, air, N2O, drive gas, and scavenging gas. In a third example of the system, optionally including one or both of the first and second examples, the module coupler does not include pneumatic components or valves. In a fourth example of the system, optionally including one or more or each of the first through third examples, the common gas manifold is coupled to a connection port fluidly coupled to each of flow paths. In a fifth example of the system, optionally including one or more or each of the first through fourth examples, the inlet module is positioned at a downstream end of the common gas manifold and the breathing system is positioned at an upstream end of the common gas manifold.

The disclosure also provides support for a manifolded anesthesia ventilator system, comprising: a common gas manifold comprised of a plurality of flow paths, wherein each flow path of the plurality of flow paths is configured to direct a gas or mixture of gases, one or more modules fluidly coupled to the common gas manifold to receive the gas or mixture of gases, the one or more modules including an inlet module, a mixer module, a vaporizer module, gas monitor module, and a breathing system. In a first example of the system, the common gas manifold is a continuous part and the one or more modules are directly fixedly coupled to the common gas manifold. In a second example of the system, optionally including the first example, the plurality of flow paths of the common gas manifold are integrated into each of the one or more modules. In a third example of the system, optionally including one or both of the first and second examples, the plurality of flow paths are each independent flow paths. In a fourth example of the system, optionally including one or more or each of the first through third examples, the common gas manifold includes a connection port comprising ports coupled each of the plurality of flow paths. In a fifth example of the system, optionally including one or more or each of the first through fourth examples, the common gas manifold and one or more modules comprise a core of the manifolded anesthesia ventilator system, and wherein the core is encased in a kernel of the manifolded anesthesia ventilator system, the kernel including system connections, a display, an inlet, and an outlet.

In an alternate embodiment, the disclosure also provides support for a manifolded anesthesia ventilator system, comprising: a first module including a first section of parallel flow paths of a backbone manifold, each of the parallel flow paths configured to direct a gas or mixture of gasses, and a second module including a second section of parallel flow paths of the backbone manifold, the second module is bolted to the first module, wherein: the first section of the parallel flow paths is fluidly coupled to the second section of the parallel flow paths, the first module is one of an inlet module, a mixer module, a vaporizer module, a gas monitor module, or a breathing system, and the second module is one of the inlet module, the mixer module, the vaporizer module, or the breathing system. In a first example of the system, the system further comprises: an upstream end cap coupled to an upstream end of the backbone manifold and a downstream end cap coupled to a downstream end of the backbone manifold. In a second example of the system, optionally including the first example, the first module and second module are not fluidly coupled by tubing.

As used herein, an element or step recited in the singular and preceded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

FIGS. 13 -15 show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A manifolded anesthesia ventilator system (200), comprising:
a core (201) including a common gas manifold (204) and one or more modules (203);
a kernel (1300) encasing the core (201) and including inputs (1308) and outputs (1309) fluidly coupled to the core.

2. The manifolded anesthesia ventilator system of claim 1, wherein the common gas manifold includes a plurality of flow paths and the one or more modules include an inlet module, mixer module, and breathing system.

3. The manifolded anesthesia ventilator system of claim 2, wherein the one or more modules further include one or more of a vaporizer module and gas monitor module.

4. The manifolded anesthesia ventilator system of claim 1, further comprising a shell coupled to the kernel, wherein the shell includes one or more of a shelf unit, drawers, a display, and a user interface.

5. The manifolded anesthesia ventilator system of claim 1, wherein the core is in a backbone configuration and the common gas manifold is spaced away from the one or more modules.

6. The manifolded anesthesia ventilator system of claim 5, wherein the common gas manifold is a continuous part and the one or more modules are directly fixedly coupled to the common gas manifold.

7. The manifolded anesthesia ventilator system of claim 1, wherein the core is in a stacked configuration and the common gas manifold is integrally formed in the one or more modules.

8. The manifolded anesthesia ventilator system of claim 1, wherein the kernel is laterally symmetric and components of a shell coupled to the kernel may be laterally interchangeable with respect to sides of the kernel.

9. The manifolded anesthesia ventilator system of claim 1, wherein a width of the kernel may be slightly wider than a width of two size E gas cylinders and a height of the kernel is slightly higher than a height of a size E gas cylinder.

10. A product line of a manifolded anesthesia ventilator system (200), the product line comprising:
a first product comprising an inlet module (202), a mixer module (206), a vaporizer module (208), a gas monitor module (210), and a breathing system (212), each of the inlet module (202), the mixer module (206), the vaporizer module (208), the gas monitor module (210), and the breathing system (212) fluidly coupled to a common gas manifold (204); and
a second product comprising the inlet module (202), the mixer module (206), the gas monitor module (21), a module coupler (402), and the breathing system (212), each of the inlet module (202), the mixer module (206), the module coupler (402), the gas monitor module (21) and the breathing system (212) fluidly coupled to the common gas manifold (204),
wherein the common gas manifold (204) is comprised of a plurality of flow paths (702), and wherein each of the plurality of flow paths (702) are configured to direct a gas or mixture of gases.

11. The product line of claim 9, wherein a position of the vaporizer module on the common gas manifold in the first product is the same as a position of the module coupler on the common gas manifold in the second product.

12. The product line of claim 9, wherein the gas or mixture of gases includes one or more of oxygen, air, N₂O, drive gas, and scavenging gas.

13. The product line of claim 9, wherein the module coupler does not include pneumatic components or valves.

14. The product line of claim 9, wherein the common gas manifold is coupled to a connection port fluidly coupled to each of flow paths.

15. The product line of claim 9, wherein the inlet module is positioned at a downstream end of the common gas manifold and the breathing system is positioned at an upstream end of the common gas manifold.
